# EUROPÄISCHE PATENTANMELDUNG

(11) **EP 3 861 920 A1**
(43) Veröffentlichungstag der Anmeldung: **11.08.2021**
(21) Anmeldenummer: 20155627.1
(22) Anmeldetag: 05.02.2020
(51) Int. Cl.: A61B 1/018, A61B 5/06

(54) **CHIRURGISCHES INSTRUMENT MIT EINER POSITIONSERKENNUNGSEINRICHTUNG**

(71) Anmelder: Erbe Elektromedizin GmbH, 72072 Tübingen (DE)
(72) Erfinder: FISCHER, Klaus, 72202 Nagold (DE); NEUGEBAUER, Alexander, 72116 Moessingen (DE); JAEGER, Jan, 72074 Tuebingen (DE); SEITZ, Bjoern, 72793 Pfullingen (DE)
(74) Vertreter: Rüger Abel Patentanwälte PartGmbB

(57) **Zusammenfassung**

Es ist ein chirurgisches Arbeitsinstrument (3) offenbart, das in einen Arbeitskanal (7) eines Endoskops (2) eingeführt und darin verschiebbar aufgenommen wird. Eine Einrichtung (27) zur Erkennung der relativen Position des Arbeitsinstrumentes (3) zu dem Endoskop (2) ist dazu eingerichtet, das Erreichen eines distalen Endes (8) des Arbeitskanals (7) durch ein distales Ende (8) des Arbeitsinstrumentes (3) in optischer Weise zu erkennen. Die Positionserkennungseinrichtung (27) weist einen Lichtleiter (28) auf, der an dem Arbeitsinstrument (3) verankert und eingerichtet ist, um Licht, das das Arbeitsinstrument (3) in der Nähe seines distalen Endes (14) umgibt, aufzunehmen, wobei über das durch den Lichtleiter (28) aufgenommene Licht die relative Position des Lichtleiters (28) und somit des Arbeitsinstrumentes (3) zu dem Endoskop (2) bestimmbar ist.

## Beschreibung

Die vorliegende Erfindung betrifft ein chirurgisches Instrument zur Einführung in einen Arbeitskanal eines Endoskops mit einer Einrichtung zur Erkennung der relativen Position des Arbeitsinstrumentes zu dem Endoskop.

Aus der DE 41 39 029 C2 ist eine Vorrichtung zur Koagulation biologischer Gewebe, insbesondere im Gastrointestinaltrakt (Magen-Darm-Trakt), bekannt, bei der sich in einem Arbeitskanal eines Endoskops eine Verbindungsleitung zum Anschluss an eine HF-Spannungsquelle befindet. Durch den Arbeitskanal wird ionisierbares Gas, z.B. Argon, zugeführt, welches am distalen Ende des Arbeitskanals austritt. Im Strömungsweg des Gases vor dem Austritt aus der Austrittsöffnung ist eine Elektrode vorgesehen, welche dem Ionisieren des Gases und der Zufuhr des Koagulationsstromes dient. Das ionisierende Gas ermöglicht die Entstehung eines Funkenüberschlags (Lichtbogens) zwischen der Elektrode und der Gewebeoberfläche, wobei durch die dabei entstehende Hitze erkranktes Gewebe bis zu wenige Millimeter tief verschorft wird.

In der vorbekannten Argon-Plasma-Koagulations(APC)-Vorrichtung ist die aktive Elektrode im definierten Abstand zu dem Endoskopende fest angeordnet. Eine übermäßige thermische Belastung des Endoskopendes durch die entstehende Hitze kann somit ausgeschlossen werden. Es sind jedoch auch APC-Vorrichtungen mit einer beweglichen APC-Sonde als Arbeitsinstrument bekannt, die in den Arbeitskanal eines Endoskops eingeführt und darin bis zu der gewünschten Arbeitsposition vorgeschoben wird, in der die aktive Elektrode geringfügig über das distale Ende des Endoskops hinausragt. Ein gewisser Mindestabstand zwischen der Elektrode und dem distalen Ende des Endoskops ist wichtig, um zu vermeiden, dass das distale Ende des Endoskops und daran befestigte Komponenten, z.B. eine Beobachtungsoptik oder CCD-Kamera, eine Beleuchtungseinrichtung für den behandelten Bereich und dgl., durch die bei der APC-Koagulation entstehende Hitze unzulässig thermisch belastet und beschädigt werden. Hierzu ist eine Einrichtung zur Erkennung der relativen Position der Elektrode zu der Endoskopspitze hilfreich.

Auch für andere Endoskopieanwendungen mit einem einführbaren Arbeitsinstrument, das eine optische Faser aufweist, wie bspw. zur optischen Emissionsspektroskopie (OES), Videoendoskopie, für starre Laparoskope, Boroskope, Fiberoskope und andere Endoskope zur Zuführung von Instrumenten in das Innere eines menschlichen Körpers, könnte eine Positionserkennung für das Arbeitsinstrument hilfreich sein, um z.B. die optische Faser in einem gewünschten Abstand zu dem distalen Ende des Endoskops zu positionieren und eine Kontamination der optischen Faser durch Flüssigkeit, Blut oder Gewebepartikelreste beim Kontakt mit dem Gewebe, z.B. der Schleimhaut eines Hohlorgans, zu vermeiden. Eine derartige Kontamination der optischen Faser kann zur Streuung, Absorption oder einer Verschlechterung der Transmission des Lichtes führen und dadurch die Endoskopiesitzung oder -behandlung beeinträchtigen.

Aus der DE 197 31 931 A1 ist ein Endoskop mit einem eingesetzten Arbeitsinstrument zur Argon-Plasma-Koagulation bekannt, das gemeinsam mit der Elektrode innerhalb des Endoskops verschiebbar ist. Um das Arbeitsinstrument sowohl für eine Kontaktkoagulation als auch für eine Nichtkontaktkoagulation und/oder zum Schneiden zu verwenden und dabei die Gefahr von Verletzungen oder Veränderungen des vorhandenen Gewebes zu reduzieren, ist im Bereich des distalen Endes ein Sensor vorgesehen, der überwacht, ob sich die aktive Elektrode des Arbeitsinstrumentes innerhalb oder außerhalb des Endoskopkatheters befindet. Als Sensor kann ein federbelasteter Mikroschaltkontakt an dem distalen Ende des Arbeitsinstrumentes, eine Miniaturlichtschranke, deren Strahl bei Anwesenheit der aktiven Elektrode unterbrochen wird, oder ein Platten-, Koaxial- oder Zylinderkondensator verwendet werden, dessen Dielektrikum durch die Anwesenheit der aktiven Elektrode beeinflusst wird. Der Nichtkontaktmodus ist nur dann aktivierbar, wenn sich die aktive Elektrode innerhalb des Endoskopkatheters befindet.

Die Positionsbestimmung erfordert zusätzliche Bauelemente, die an dem distalen Ende des Arbeitsinstrumentes vorzusehen sind, Verbindungen zu dem distalen Ende, um dem Sensor elektrische oder optische Messsignale zuzuführen, und eine geeignete Auswertung der durch den Sensor gelieferten Signale. Der Aufwand für die Implementierung und Auswertung ist beachtlich.

DE 10 2004 039 202 B3 beschreibt eine Vorrichtung zur Messung einer relativen Position eines chirurgischen Arbeitsinstrumentes, wobei die Positionsbestimmung über eine Messung des komplexen Widerstands zwischen dem Arbeitsinstrument und dem Arbeitskanal eines Endoskops oder alternativ über ein pneumatisches oder akustisches Messsystem erfolgt. Zur Messung des komplexen Widerstands muss das Endoskop oder zumindest dessen Arbeitskanal elektrisch leitend ausgebildet werden. Für eine akustische oder pneumatische Messung muss ein Messsignal in Form eines Schallsignals oder eines Gleich- oder Wechseldruckes eines Gases erzeugt und entweder in den Arbeitskanal des Endoskops oder in ein Lumen des Arbeitsinstrumentes eingekoppelt und an dem anderen von dem Arbeitskanal und dem Lumen über einen elektromechanischen Wandler oder einen Druckaufnehmer erfasst werden. Dies kann relativ aufwendig sein.

Es besteht weiterhin ein Bedarf nach einer einfachen Erkennung der Position eines Arbeitsinstrumentes relativ zu einem Arbeitskanal eines Endoskops, in den das Instrument eingeführt wird.

Ausgehend hiervon ist es eine Aufgabe der vorliegenden Erfindung, eine Erkennungseinrichtung für die relative Position eines chirurgischen Arbeitsinstrumentes zu einem Arbeitskanal eines Endoskops mit einfachen Mitteln und geringem Aufwand für die Implementierung und Auswertung zu schaffen.

Insbesondere ist es eine Aufgabe der Erfindung, ein chirurgisches Instrument zur Einführung in einen Arbeitskanal eines Endoskops, in dem das Arbeitsinstrument verschiebbar angeordnet wird, mit einer Einrichtung zur Erkennung der relativen Position des Arbeitsinstrumentes zu dem Endoskop zu schaffen, die eine Einstellung der für die jeweilige Anwendung gewünschten Arbeitsposition des Arbeitsinstrumentes relativ zu dem Endoskop mit einfachen Mitteln, bei geringem Aufwand für die Implementierung und Auswertung ermöglicht, so dass eine thermische Beeinträchtigung des distalen Endes des Endoskops, eine Verschmutzung einer optischen Faser und/oder eine Gefahr für den Patienten oder Anwender vermieden oder zumindest stark reduziert werden kann.

Zur Lösung dieser Aufgabe ist gemäß der Erfindung das chirurgische Arbeitsinstrument mit den Merkmalen des Anspruchs 1 geschaffen.

Das chirurgische Arbeitsinstrument zur Einführung in einen Arbeitskanal eines Endoskops, in dem das Arbeitsinstrument verschiebbar angeordnet wird, ist mit einer Einrichtung zur Erkennung der relativen Position des Arbeitsinstrumentes zu dem Endoskop versehen, wobei die Positionserkennungseinrichtung einen Lichtleiter aufweist, der an dem Arbeitsinstrument verankert und eingerichtet ist, um Licht, das das Arbeitsinstrument in der Nähe eines distalen Endes umgibt, aufzunehmen, wobei über das durch den Lichtleiter aufgenommene Licht die relative Position des Arbeitsinstrumentes zu dem Endoskop bestimmbar ist.

Die Erfindung beruht auf der Erkenntnis, dass in das Arbeitsinstrument ein Lichtleiter derart eingearbeitet und an diesem verankert werden kann, dass er beim Einführen des Arbeitsinstrumentes in den Arbeitskanal eines Endoskops gemeinsam mit diesem eingeschoben und darin verschoben wird, wobei der Lichtleiter derart angeordnet ist, dass er direkt oder indirekt das Licht am distalen Ende empfängt, und wobei über das Licht die relative Position des Arbeitsinstrumentes erkannt werden kann. Über die Art, Stärke, Frequenz, Anwesenheit und/oder Abwesenheit des aufgenommenen Lichts kann bestimmt werden, ob und gegebenenfalls wie weit das Arbeitsinstrument in den Arbeitskanal des Endoskops eingeschoben ist und ob es die gewünschte Arbeitsposition erreicht hat. Die Positionserkennung arbeitet berührungslos und erfordert keine speziellen elektrischen oder mechanischen Bauelemente, insbesondere keine Kontaktschalter, an dem distalen Ende des Endoskops. Es müssen auch keine speziellen Messsignale, wie Strom-, Spannungs-, optische, akustische oder pneumatische Signale, gezielt erzeugt und durch das Arbeitsinstrument hindurch eingeleitet werden, um die Auswertung der relativen Position zu ermöglichen. Es ist auch keine Modifikation des Endoskops erforderlich.

Vielmehr kann der Lichtleiter einfach derart an dem Arbeitsinstrument angeordnet werden, dass die Position des Lichtleiters, die über das aufgenommene Licht ermittelt wird, die Position des Arbeitsinstrumentes kennzeichnet.

Bevorzugterweise kann der Lichtleiter eine kostengünstige optische Faser, insbesondere eine Glasfaser oder eine auf Kunststoff basierende optische Faser, z.B. eine polymere optische Faser, aufweisen.

Der Lichtleiter kann sich ausgehend von einer Stelle in der Nähe des distalen Endes des Arbeitsinstrumentes bis zu einem proximalen Ende des Arbeitsinstrumentes erstrecken, um das durch den Lichtleiter aufgenommene Licht zur Auswertung zu dem proximalen Ende des Arbeitsinstrumentes zu übertragen.

In bevorzugten Ausführungsformen der Erfindung weist das Arbeitsinstrument ein Rohr, insbesondere ein starres Rohr oder vorzugsweise einen flexiblen Schlauch, auf, das bedarfsweise, jedoch nicht notwendigerweise aus einem lichtundurchlässigen Material bestehen kann, wobei die Positionserkennungseinrichtung eine Lichteinleitfläche aufweist, die an der Außenseite des Rohrs angeordnet und zur seitlichen Lichterfassung des das distale Ende des Arbeitsinstrumentes umgebenden Lichtes eingerichtet ist. Dadurch wird im Inneren des Arbeitskanals des Endoskops eine wenigstens nahezu vollständige Abdunkelung erzielt, bis das Arbeitsinstrument diesen verlässt, was eine gute Detektierbarkeit des Austritts des Arbeitsinstrumentes aus dem Arbeitskanal ermöglicht.

Prinzipiell ist es alternativ auch möglich, das Licht in axialer Richtung zu erfassen, wobei die seitlich gerichtete Lichterfassung aber eine leichtere und besser abgestimmte, genauere Positionserkennung ermöglicht.

In einer bevorzugten Ausführungsform erstreckt sich der Lichtleiter, z.B. die optische Faser, im Wesentlichen in dem inneren Kanal (Lumen) des Rohrs des Arbeitsinstrumentes entlang dessen Längserstreckung, wobei die Lichteinleitfläche durch eine aus dem Rohr seitlich herausgeführte Stirnfläche des Lichtleiters gebildet ist. Die Realisierung ist einfach, und der Lichtleiter ist im Inneren des Rohrs geschützt untergebracht. Der Lichtleiter kann vorzugsweise in dem inneren Kanal des Rohrs frei verlaufen oder wenigstens abschnittsweise an der Innenseite des inneren Kanals befestigt sein.

In einer weiteren Ausführungsform ist der Lichtleiter im Wesentlichen an der Außenseite des Rohrs angebracht und erstreckt sich entlang dessen Längserstreckung von dem distalen Ende bis zu dem proximalen Ende des Rohrs. Die Lichteinleitfläche kann durch eine abgeschrägte Stirnfläche (Faserfacette) des Lichtleiters gebildet sein. Die Realisierung ist ebenfalls einfach, wobei die Anbringung des Lichtleiters, z.B. der optischen Faser, an der Außenseite des Rohrs sich einfacher gestaltet.

In einer weiteren Ausführungsform ist die Lichteinleitfläche durch einen länglichen Glasstab gebildet, der von dem Lichtleiter gesondert ist. Der Glasstab, der vorzugsweise aus Silikatglas oder Acrylglas besteht, ist an der Außenseite des Rohrs befestigt und weist eine bestimmte Länge auf, die größer als die Breite des Glasstabs ist. Die Länge kann größer als die halbe Auskraglänge des distalen Endes des Arbeitsinstrumentes in der gewünschten Arbeitsposition sein. Der Glasstab dient der Erfassung des umgebenden Lichts an der Außenseite und der Leitung des erfassten Lichts zu dem Lichtleiter, der mit einem dem distalen Ende des Arbeitsinstrumentes abgewandten Ende des Glasstabs zur Lichtaufnahme und -übertragung verbunden ist.

Durch die vergrößerte Ausdehnung des Glasstabs in Längsrichtung des Rohrs ist es möglich, unterschiedliche Mengen oder Intensitäten des umgebenden Lichts zu erfassen, je nachdem, wie weit der Glasstab über das distale Ende des Endoskops nach außen ragt und so dem Licht ausgesetzt ist. Damit ist eine Feinbestimmung der Positionen des Arbeitsinstrumentes möglich.

In jeder der Ausführungsformen könnte der Lichtleiter auch bereits bei der Extrusion eines PTFE-Schlauchs des Arbeitsinstrumentes wahlweise an der Innen- oder Außenseite des Schlauchs oder in die Schlauchwand eingebettet werden.

Unabhängig von der Ausführungsform ist die erfindungsgemäße Positionserkennungseinrichtung, abgesehen von dem Lichtleiter und ggfs. dem gesonderten Lichterfassungselement, wie bspw. dem Glasstab, frei von Linsen, Spiegeln oder sonstigen optischen Elementen zum Umleiten des in Bezug auf die Längsachse des Arbeitsinstrumentes radial oder seitlich erfassten Lichtstrahls zu dem längs des Arbeitsinstrumentes verlaufenden Lichtleiter. Somit kann die Positionserkennungseinrichtung mit wenigen und relativ einfachen, kostengünstigen Mitteln verwirklicht werden, so dass sie sich auch für Arbeitsinstrumente, wie bspw. Endoskopsonden, eignet, die für den einmaligen Gebrauch bestimmt sind und nach dem Gebrauch entsorgt werden müssen.

Das chirurgische Arbeitsinstrument kann ferner eine Auswerteeinrichtung aufweisen, die an dem proximalen Ende des Arbeitsinstrumentes anschließbar oder angeschlossen und eingerichtet ist, um das durch den Lichtleiter aufgenommene und geleitete Licht zu empfangen und auszuwerten, um die relative Position zu ermitteln.

In vorteilhaften Ausführungsformen kann die Auswerteeinrichtung eine optische Spektrometereinheit aufweisen, die eingerichtet ist, um das aufgenommene Licht in seine spektralen Bestandteile aufzuteilen, um das Spektrum des aufgenommenen Lichts, also die Intensität des Lichts über einem Bereich von Lichtfrequenzen oder in bestimmten diskreten Frequenzen bestimmen zu können. Die Spektrometereinheit kann bspw. auf einem lichtbrechenden Prisma oder lichtbeugenden Gitter basieren. Die Auswerteinheit kann ferner einen elektro-optischen Wandler, ein Fotodiodenarray oder eine sonstige geeignete Vorrichtung aufweisen, um das spektral aufgeteilte Licht in entsprechende elektrische Signale umzuwandeln, die ausgewertet oder analysiert werden können, um das Frequenzspektrum des aufgenommenen Lichtsignals zu bestimmen. Die Frequenzspektrumanalyse kann auch z.B. mit Hilfe einer Fourier-Analyse erfolgen. Es kann ein kontinuierliches Frequenzspektrum für im Wesentlichen den gesamten Frequenzbereich des aufgenommenen Lichtes, nur in einzelnen Frequenzbändern oder auch nur in vorbestimmten diskreten Frequenzen ermittelt werden.

Die Auswerteeinrichtung kann ferner eine Spektrumanalyseeinheit aufweisen, die eingerichtet ist, um die ermittelte Intensität des Lichts über einem Bereich von Lichtfrequenzen oder in bestimmten diskreten Frequenzen mit abgespeicherten Intensitätswerten von Referenzspektren zu vergleichen und anhand des Vergleichs das durch den Lichtleiter aufgenommene Licht zu klassifizieren oder zu identifizieren. Insbesondere kann ein Referenzspektrum des Beleuchtungslichts, bspw. einer Kaltlichtquelle, des Endoskops vorab abgespeichert werden, um bei der Auswertung durch die Spektrumanalyseeinheit verwendet zu werden. Ferner kann wenigstens ein allgemeines Referenzspektrum für ein Umgebungslicht in einem Behandlungsraum, in dem das Endoskop mit dem chirurgischen Arbeitsinstrument verwendet wird, vorgegeben und abgespeichert werden. Außerdem besteht die Möglichkeit, das tatsächliche Umgebungslicht in dem Behandlungsraum bei der Inbetriebnahme, der Kalibrierung oder einer Instandhaltung eines chirurgischen Gerätes zu erfassen, in ein zugehöriges Referenzspektrum umzuwandeln und dieses für die nachfolgenden Analysen durch die Auswerteeinrichtung abzuspeichern. Jedenfalls können Intensitätsverhältnisse in verschiedenen charakteristischen Frequenzbereichen, -bändern oder -linien für das durch den Lichtleiter aufgenommene Licht im Vergleich zu den vorgegebenen, abgespeicherten Referenzspektren bestimmt werden.

Die Spektrumanalyseeinheit kann dann eingerichtet sein, um wenigstens zwischen einem Behandlungsraum-Umgebungslicht, das erfasst wird, wenn das chirurgische Arbeitsinstrument sich außerhalb des Endoskops befindet, einer wenigstens nahezu völligen Dunkelheit, die erfasst wird, wenn das chirurgische Arbeitsinstrument in den Arbeitskanal des Endoskops eingeführt ist, aber mit seinem distalen Ende noch nicht aus dem Arbeitskanal des Endoskops herausragt, und einem Behandlungslicht oder Operationslicht aus dem Endoskop zu differenzieren, das erfasst wird, wenn das distale Ende soweit aus dem Arbeitskanal des Endoskops heraus und in den behandelten Bereich im Innern eines Patienten hinein ragt, dass über die Lichteinleitfläche und den Lichtleiter das durch das Endoskop bereitgestellte Behandlungs- oder Operationslicht erfasst wird.

Die Spektrumanalyseeinheit kann somit eingerichtet sein, um zwischen mehreren Lichtquellen, insbesondere der Kaltlichtquelle eines Endoskops, einem Umgebungslicht und bedarfsweise auch dem von einem Plasma emittierten Licht bei der Koagulation zu unterscheiden. Während Umgebungslicht Wellenlängen im sichtbaren Bereich, also zwischen etwa 400 nm und 800 nm, aufweist, wird das Arbeitslicht einer Kaltlichtquelle des Endoskops im Allgemeinen ein charakteristisches Spektrum im Bereich von 400-700 nm haben, während das von einem Plasma emittierte Licht charakteristische Spektralanteile zwischen 200 und 400 nm, aber auch Anteile im sichtbaren Frequenzbereich zeigt. Die differierenden charakteristischen Spektralverläufe, -anteile oder -linien ermöglichen eine zuverlässige Unterscheidbarkeit der erfassten Lichtarten durch die Spektrumanalyseeinheit.

Die Auswerteeinrichtung kann ferner eine Detektoreinheit aufweisen, die eingerichtet ist, um beim Einschieben des Arbeitsinstrumentes in den Arbeitskanal des Endoskops zu erkennen, wenn die Lichteinleitfläche der Positionserkennungseinrichtung der Umgebung eines Behandlungsraums ausgesetzt ist, aus der Umgebung des Arbeitsraums in ein proximales Ende des Arbeitskanals eintritt oder aus dem Arbeitskanal in einen zu behandelnden Körperbereich heraustritt, und basierend auf dieser Erkennung entsprechende Positionserkennungssignale zu generieren. Die Positionserkennungssignale können dann zur weiteren Verwertung zu einer übergeordneten Steuerung für das chirurgische Arbeitsinstrument und/oder das Endoskop übertragen werden.

In einer besonders bevorzugten Ausführungsform äußerst geringer Komplexität weist die Auswerteeinrichtung eine Detektoreinrichtung auf, die eingerichtet ist, um zu erkennen, ob von dem Lichtleiter Licht einer Mindestintensität aufgenommen wird oder nicht und basierend darauf Positionserkennungssignale zu generieren, die kennzeichnen können, dass sich das distale Ende des Arbeitsinstrumentes außerhalb oder innerhalb des Arbeitskanals des Endoskops befindet. Hierzu kann die Auswerteeinrichtung ein Mittel, z.B. eine Fotodiode, zur Umwandlung des aufgenommenen Lichtsignals in ein elektrisches Signal und eine Detektoreinheit (einen Hell-Dunkel-Detektor) aufweisen, die die Stärke des elektrischen Signals z.B. mit einer vorbestimmten Schwelle vergleichen kann, um festzustellen, ob das Signal Helligkeit oder Dunkelheit kennzeichnet. Es kann ein Wechsel von hell zu dunkel oder umgekehrt erfasst werden. Dies ermöglicht einer übergeordneten Steuerung oder einem Benutzer, anhand deren Kenntnisse über den momentanen Betriebsablauf festzustellen, dass sich das Arbeitsinstrument in der Behandlungsraum-Umgebung, in dem Arbeitskanal des Endoskops oder in der gewünschten Arbeitsposition befindet. Mit dem Hell-Dunkel-Sensor lassen sich bestimmte Funktionen des Arbeitsinstrumentes, wie bspw. Initiieren eines leichten Inertgasflusses (z.B. Argon) während des Einführens des Arbeitsinstrumentes in das Endoskop zu Reinhaltungszwecken oder Sperren der HF-Signale, bis sich das Instrument in der korrekten Position befindet, sehr einfach automatisieren. Eine Spektralanalyse und genauere Positionsbestimmung sind hierzu nicht erforderlich.

In besonders vorteilhaften Weiterbildungen der Erfindung kann die auf einem Hell-Dunkel-Sensor basierende Auswerteeirichtung in einem integralen Verbinder, einem Stecker oder einer Steckerbuchse, der an einem proximalen Ende des Arbeitsinstrumentes angeordnet und zum Anschluss an ein externes Steuergerät vorgesehen ist, integriert sein und direkt elektrische Signale an das Steuergerät ausgeben, die zur automatisierten Steuerung genutzt werden können. Diese Ausführungsform ist insbesondere für Einmal-Sonden gut geeignet, da die Auswerteeirichtung äußerst kostengünstig realisiert und gemeinsam mit dem Arbeitsinstrument nach einmaliger Benutzung entsorgt werden kann.

Wenn die Auswerteeinrichtung komplexer mit der Spektrometereinheit und der Spektrumanalyseeinheit ausgebildet ist, kann sie auch prozessorbasiert implementiert sein. Manche Funktionsteile der Auswerteeinrichtung, wie z.B. die Detektoreinheit, können hierbei in einem Verbinder des chirurgischen Arbeitsinstrumentes verbaut sein, der als Schnittstelle zwischen dem chirurgischen Arbeitsinstrument und einem übergeordneten Steuergerät dient, während andere Funktionsteile, die z.B. die Speicherung und Auswertung von Daten bzw. Signalen betreffen, in dem externen Steuergerät integriert sein können.

Es kann jedenfalls eine zusätzliche Steuerung für das Arbeitsinstrument und/oder das Endoskop vorgesehen sein, die mit der Auswerteeinrichtung verbunden oder verbindbar ist und die eingerichtet ist, um basierend auf den von der Detektoreinheit übertragenen Positionserkennungssignalen (im einfachsten Fall Hell-Dunkel-Sensorsignalen) automatisch bestimmte Maßnahmen einzuleiten. Bspw. kann die Steuerung bei Empfang eines ersten Positionserkennungssignales, das den Beginn des Einführens des Arbeitsinstrumentes in das Endoskop kennzeichnet, die Zuführung eines inerten Gases, z.B. Argon, zu dem Arbeitsinstrument zur Reinhaltung desselben freigegeben, falls dies erwünscht oder erforderlich ist. Außerdem kann in diesem Stadium eine Voraktivierung erfolgen, bei der das chirurgische Gerät für die anschließende Behandlung parametrisiert wird. Z.B. können bei der Anwendung zur Koagulation die erforderliche HF-Spannung und der Koagulationsstrom vorgewählt und die Behandlungs- bzw. Operationsbeleuchtung des Endoskops eingeschaltet werden. Vorzugsweise wird eine Aktivierungssperre bewirkt, die eine ungewollte Zündung einer Plasmaentladung zur Ionisierung des Gasstrahls und somit einen ungewollten elektrischen Überschlag solange verhindert, bis sich das chirurgische Arbeitsinstrument in der gewünschten Arbeitsposition in Bezug auf das Endoskop befindet. Damit kann vermieden werden, dass es aufgrund einer unmittelbaren Nähe des Endoskopendes zu der zu behandelnden Gewebeoberfläche bereits dann zu einem elektrischen Überschlag kommt, wenn sich die Elektrode des Arbeitsinstrumentes noch innerhalb des Arbeitskanals des Endoskops befindet oder nur geringfügig aus diesem herausragt, weil durch die dadurch entstehende Hitze der Körper und weitere Komponenten des Endoskops, wie bspw. Beobachtungs- und Beleuchtungseinrichtungen, beschädigt werden könnten.

Wenn die Steuerung ein weiteres Positionserkennungssignal von der Detektoreinheit empfängt, das anzeigt, dass das distale Ende des Arbeitsinstrumentes, z.B. mit der Elektrode, die gewünschte Arbeitsposition, also den für die Behandlung gewünschten Abstand zu dem distalen Ende des Endoskops erreicht hat, kann die Steuerung automatisch die Aktivierungssperre aufheben und daraufhin das Anlegen der erforderlichen HF-Spannung an die Elektrode und die Zuführung des erforderlichen Koagulationsstroms, die Zuführung des Inertgases mit dem erforderlichen Druck, falls noch nicht geschehen, die Steuerung eines Fluidflusses oder eine sonstige Steuerung des Betriebs des Arbeitsinstrumentes und/oder des Endoskops bewirken.

In der bevorzugten Anwendung ist das chirurgische Arbeitsinstrument eine Argon-Plasma-Koagulations(APC)-Sonde zur HF-Koagulation biologischer Gewebe mittels eines flexiblen Endoskops. Die Sonde weist dann ein Rohr in Form eines flexiblen Schlauchs aus einem elektrisch nicht leitenden Material, z.B. aus Polytetrafluorethylen (PTFE), durch den ein inertes Gas, insbesondere Argon, zuführbar ist, und eine am distalen Ende des Rohrs angeordnete Elektrode auf, der über eine Verbindungsleitung, die in dem inneren Kanal des Rohrs verläuft, ein HF-Koagulationsstrom zur Ionisierung des Gases zuführbar ist.

Das chirurgische Arbeitsinstrument kann auch für andere, bspw. diagnostische Endoskopiezwecke, für Videoskope, Endoskope, Gastroskope, Bronchoskope, Koloskope, Hysteroskope, Zystoskope, Arthroskope, Herzkatheter, Boroskope, Fiberskope, Loperoskope und dgl., auch für Hyperthermie-Anwendungen, etc. eingerichtet sein, wobei die Positionserkennungseinrichtung es stets ermöglicht, einen Kopf am distalen Ende des Arbeitsinstrumentes während der Behandlung, Operation oder Sitzung in der gewünschten relativen Position in Bezug auf das Endoskop zu positionieren.

Es kann auch eine Anzeigeeinrichtung vorgesehen sein, die eingerichtet ist, um die von der Detektoreinheit ausgegebenen Positionserkennungssignale in optischer, akustischer, taktiler oder sonstiger Weise einem Bediener anzuzeigen, um diesen auf die jeweilige Lage des Arbeitsinstrumentes hinzuweisen. Der Bediener kann dann manuell die jeweils erforderlichen Maßnahmen zur Steuerung eines Betriebs des Arbeitsinstrumentes bzw. Endoskops treffen.

Die Erfindung ermöglicht es, auf einfache Weise, bei geringem Realisierungs- und Auswerteaufwand, die relative Position eines chirurgischen Arbeitsinstrumentes zu dem Endoskop zu erkennen, um z.B. Beschädigungen des Endoskops während einer Koagulation zu vermeiden, zulässige thermische Belastungen an dem distalen Ende sowohl der Sonde als auch des Endoskops einzuhalten und/oder einen Kontakt mit dem zu behandelnden Gewebe mit einer Kontaminierung einer optischen Faser zu vermeiden. Durch die Lichtleitung über den Lichtleiter und die relativ schnelle Auswertung entstehen nur geringe Verzögerungszeiten. Es werden kostengünstige Komponenten verwendet, die, insbesondere bei der Verwendung des im Verbinder verbauten Hell-Dunkel-Detektors, besonders auch für Einwegsonden geeignet sind. Es werden keine Kontaktschalter, keine Lichtschranken, keine sonstigen Mittel, denen gezielt Messsignale zugeführt werden müssen, und keine Shunt-Widerstände in der Messschaltung benötigt.

Weitere vorteilhafte Einzelheiten von Ausführungsformen der Erfindung ergeben sich aus den Unteransprüchen, der Zeichnung sowie der zugehörigen Beschreibung. Die Erfindung ist nachstehend anhand einer Zeichnung näher beschrieben, die beispielhafte, keinesfalls beschränkende Ausführungsformen der Erfindung zeigt, wobei gleiche Bezugszeichen in allen Figuren verwendet werden, um gleiche Elemente zu bezeichnen. Es zeigen:
Figur 1 ein medizinisches Gerät mit einem Endoskop und darin eingeschobenem Arbeitsinstrument gemäß der Erfindung in stark schematisierter Perspektivdarstellung;
Figur 2 das Arbeitsinstrument aus Figur 1 in einer isolierten, stark schematisierten Perspektivdarstellung;
Figur 3 einen Längsschnitt durch das Arbeitsinstrument aus den Figuren 1 und 2 in vereinfachter Darstellung;
Figuren 4a und 4b Ausführungsformen von Auswerteeinrichtungen für das Arbeitsinstrument der Figuren 1-3 in vereinfachten Blockschaltbildern;
Figuren 5a-5c vereinfachte Prinzipdarstellungen des Endoskops und Arbeitsinstrumentes in unterschiedlichen relativen Stellungen zueinander zur Veranschaulichung der Funktionsweise;
Figur 6 eine weitere Ausführungsform eines Arbeitsinstrumentes gemäß der Erfindung in stark schematisierter Längsschnittdarstellung; und
Figuren 7a und 7b eine weitere Ausführungsform eines Arbeitsinstrumentes gemäß der Erfindung in stark schematisierten Längsschnittdarstellungen in unterschiedlichen relativen Positionen zu einem Endoskop.

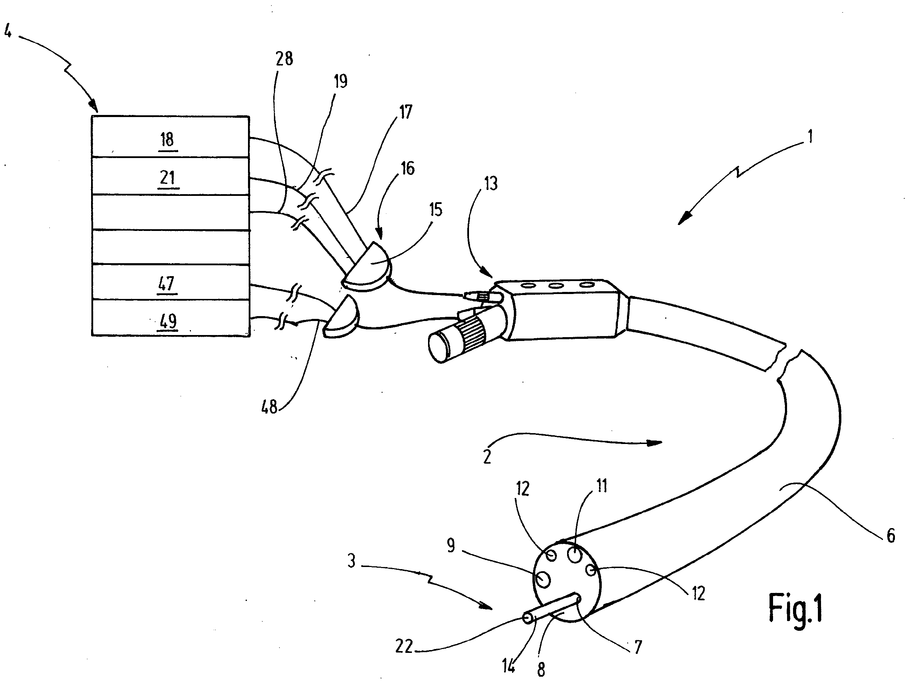

In Figur 1 ist in einer stark schematisierten Darstellung ein Endoskopsystem 1 gemäß einer Ausführungsform der Erfindung mit einem flexiblen Endoskop 2, einem in dem Endoskop 2 eingesetzten Arbeitsinstrument 3 und einem Steuergerät 4 veranschaulicht. Das Endoskopsystem 1 ist im vorliegenden Beispiel zur Durchführung einer Argon-Plasma-Koagulation (APC) zur Stillung von Blutungen aus Läsionen (Verletzungen) insbesondere des Magen-Darm-Trakts vorgesehen, wobei die Erfindung auch in anderen medizinisch-endoskopischen Systemen Verwendung finden kann. Während bspw. hier ein flexibles Endoskop 2 dargestellt ist, kann in anderen Anwendungen auch ein starres Endoskop 2 verwendet werden. Das erfindungsgemäße Endoskopsystem kann für vielfältige weitere Behandlungen oder Operationen, z.B. zur Desikkation einer Gewebeoberfläche, Erodikation von Resten nach einer Polypektomie, zur Turmorverschorfung oder zur thermischen Gewebemarkierung eingesetzt werden.

Das Endoskop 2 weist hier ein längliches, biegbares Rohr oder einen flexiblen Schlauch 6 aus einem geeigneten biokompatiblen, flexiblen Material, insbesondere Polymermaterial, wie bspw. Polytetrafluorethylen (PTFE), auf, das sich zur Verwendung im Inneren eines Patientenkörpers eignet. Der Schlauch 6 definiert einen Arbeitskanal 7, in den unterschiedliche Arbeitsinstrumente eingesetzt werden können. Bspw. ist das chirurgische Arbeitsinstrument 3 hier in dem Arbeitskanal 7 des Endoskops 1 verschiebbar aufgenommen, wobei es aus einem distalen Ende 8 des Endoskops 2, das zur Einführung in einen Patientenkörper bestimmt ist, aus dem Arbeitskanal 7 nach außen ragt. An dem distalen Ende 8 ist ferner ein optionaler weiterer Arbeitskanal 9 herausgeführt, der bedarfsweise für andere Arbeitsinstrumente verwendet werden kann.

Ferner ist an dem distalen Ende 8 des Endoskops 2 eine Linse oder Beobachtungseinrichtung 11 angeordnet, die eine CCD-Kamera umfassen oder über einen Glasfaserstrang mit einer hier nicht dargestellten Okulareinrichtung verbunden sein kann.

Außerdem enthält das Endoskop 2 eine Beleuchtungseinrichtung 12 mit Leuchtmitteln, die an dem distalen Ende 8 des Endoskops 1 nach außen ragen und über einen Lichtwellenleiter mit einer Lichtquelle, vorzugsweise Kaltlichtquelle, verbunden sind, um im Einsatz Licht hoher Intensität im sichtbaren Spektralbereich zu empfangen und auf einen hier nicht näher dargestellten Behandlungs- oder Operationsbereich im Inneren eines Patienten zu richten. Dadurch wird eine Beobachtung des Behandlungs- oder Operationsbereichs über die Beobachtungseinrichtung 11, einschließlich eine Aufnahme von Bildern über eine CCD-Kamera, ermöglicht, während durch das Kaltlicht eine Hitzeentwicklung wie bei gewöhnlichen Lichtquellen vermieden wird.

Das Arbeitsinstrument 3 kann in ein proximales Ende 13 des Endoskops 2 soweit eingeschoben werden, bis es, wie in Figur 1 veranschaulicht, aus dem distalen Ende 8 des Arbeitskanals 7 hervortritt. In dieser Position befindet sich ein distales Ende 14 des Arbeitsinstrumentes 3 in dem durch die Beleuchtungseinrichtung 12 beleuchtbaren Blickfeld der Beobachtungseinrichtung 11 des Endoskops 2.

Ein proximales Ende 16 des Arbeitsinstrumentes 3 ist über einen Verbinder oder Stecker 15 an das Steuergerät 4 angeschlossen. Der Verbinder 15 umfasst eine Gaszufuhrleitung 17, über die das Arbeitsinstrument 3 mit einer Gasversorgungseinrichtung 18 des Steuergeräts 4 in Strömungsverbindung steht. Die Gasversorgungseinrichtung 18 ist mit einem Gasvorrat, bspw. einer Argon enthaltenden Gasflasche, verbunden und dazu eingerichtet, das Gas, z.B. Argon, mit einem vorgegebenen, für die jeweilige Behandlung geeigneten Druck dem Arbeitsinstrument 3 zuzuführen.

Der Verbinder 15 umfasst ferner eine elektrische Verbindungsleitung 19, über die das Arbeitsinstrument 3 mit einer Hochfrequenz(HF)-Generatoreinrichtung 21 des Steuergerätes 4 elektrisch leitend verbunden ist, um von dieser eine erforderliche HF-Spannung zur Zündung einer Plasmaentladung zur Ionisierung des inerten Gases, z.B. Argons, zur Zuführung von Koagulationsstrom zu dem Gewebe zu erhalten. Hierzu ist an dem distalen Ende 14 des Arbeitsinstrumentes 3 eine Elektrode 22 vorgesehen, die an die Verbindungsleitung 19 angeschlossen ist. Die Elektrode 22 ist hier als eine stiftförmige oder rohrförmige Zündelektrode dargestellt, kann aber beliebige Formen, bspw. die eines Zündplättchens, annehmen. Die Elektrode 22 kann auch in einem hier nicht näher dargestellten Ansatzstück oder Kopfteil des Arbeitsinstrumentes 3 angeordnet sein, das bspw. aus Keramik ausgebildet sein kann, um der Hitzeeinwirkung aufgrund der gezündeten Plasmaentladung im Betrieb zu widerstehen.

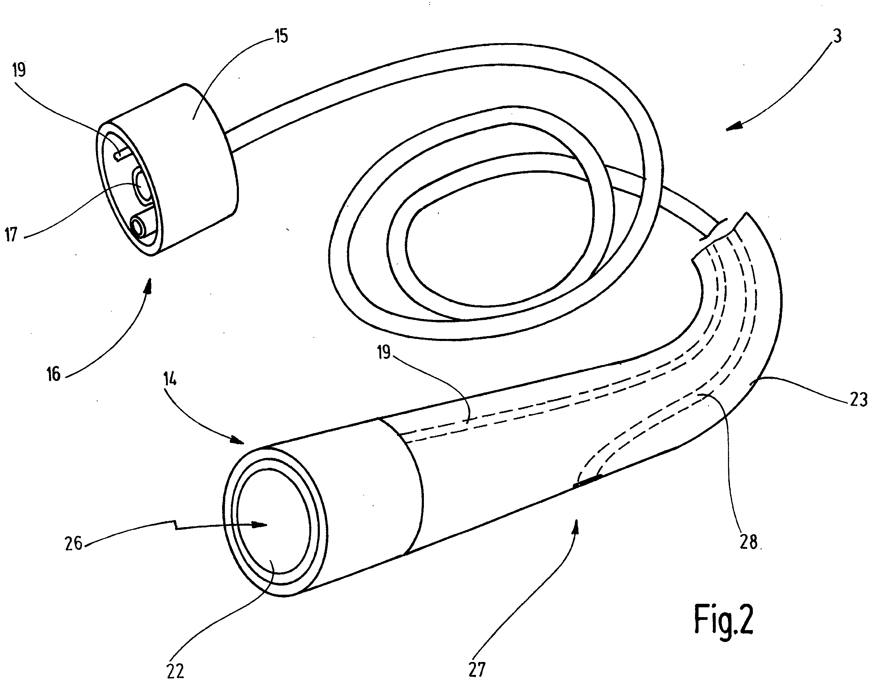

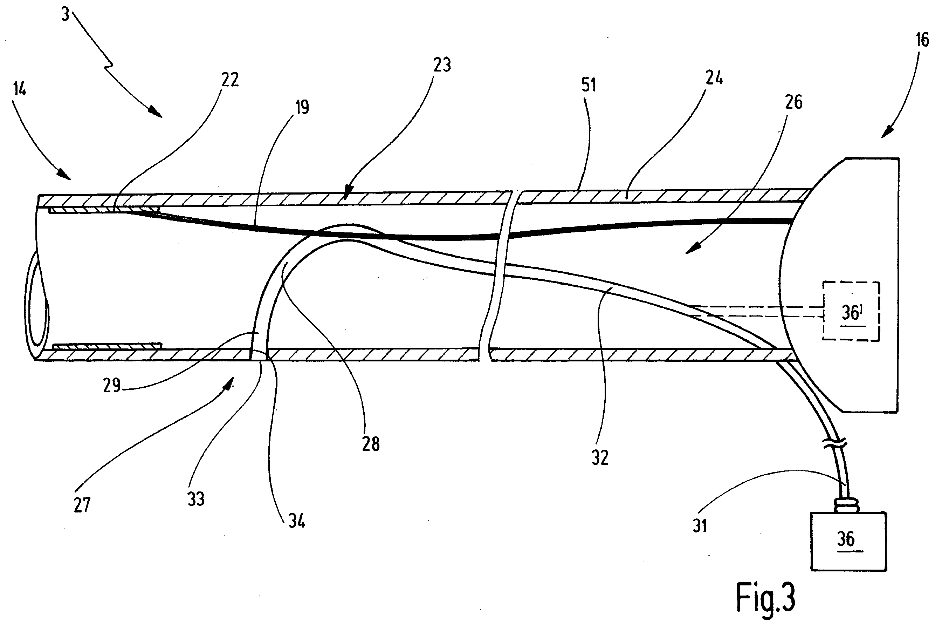

Das chirurgische Arbeitsinstrument 3 ist in Figur 2 in einer isolierten, vereinfachten Perspektivdarstellung und in Figur 3 in schematisierter Längsschnittdarstellung gezeigt. Wie veranschaulicht, weist das Arbeitsinstrument 3, das auch als eine Sonde bezeichnet wird, ein Rohr 23 auf, das hier in Form eines flexiblen Schlauchs ausgebildet ist und aus einem geeigneten, biokompatiblen Material, das sich für die vorliegenden Anwendungen eignet, insbesondere aus PTFE, ausgebildet ist. Prinzipiell könnte bei einem starren Endoskop auch ein starres Rohr 23 verwendet werden. Das Rohr 23 weist eine Wand 24 auf, die einen im Wesentlichen zylindrischen, auch als Lumen bezeichneten inneren Kanal 26 definiert, durch den dem distalen Ende 14 des Arbeitsinstrumentes 3 das Gas, z.B. Argon, zugeführt wird. In dem inneren Kanal 26 ist auch die Verbindungsleitung 19 untergebracht, die sich ausgehend von dem proximalen Ende 16 des Arbeitsinstrumentes 3 bis zu dessen distalem Ende 14 erstreckt und dort mit der Elektrode 22 verbunden ist.

Es ist bei der Argon-Plasma-Koagulation wichtig, die Elektrode 22 des Arbeitsinstrumentes 3 im Einsatz an einer geeigneten relativen Position in Bezug auf das Endoskop 2 zu positionieren. Insbesondere sollte die Elektrode 22 in einem Mindestabstand zu dem distalen Ende 8 des Endoskops 2 positioniert werden, damit nach dem Zünden der Plasmaentladung, wenn das ionisierte Gas als ein Leiter zur Zuführung des Koagulationsstroms dient, die dabei entstehende Hitze das Endoskop 2 nicht beeinträchtigt. Bspw. kann das Arbeitsinstrument 3 zum Einmalgebrauch bestimmt sein, so dass es relativ kostengünstig hergestellt ist und nach einer endoskopischen Behandlung oder Operation weggeworfen wird. Hingegen ist das Endoskop mit seinen Komponenten relativ kostspielig und zur Wiederverwendung bestimmt. Deshalb müssen insbesondere an dem distalen Ende 8 des Endoskops 2 angeordnete Komponenten, wie bspw. die Beobachtungseinrichtung 11, die Beleuchtungseinrichtung 12 und dgl., gegen Beschädigung durch die im Betrieb entwickelte Hitze geschützt werden. Hierzu ist bspw. bei einer APC-Sonde 3 mit einem Außendurchmesser von etwa 2 - 5 mm ein Mindestabstand zwischen der APC-Sondenspitze mit der Elektrode 22 und dem distalen Ende 14 des Endoskops 2 von wenigstens etwa 10 mm erforderlich.

Umgekehrt sollte die Elektrode 22 aber auch nicht zu weit aus dem Arbeitskanal 7 des Endoskops 2 herausragen, damit sie im Betrieb im Beleuchtungsfeld der Beleuchtungseinrichtung 12 und im Blickfeld der Beobachtungseinrichtung 11 angeordnet ist und auch unerwünschte Berührungen der zu behandelnden Gewebeoberfläche durch die Elektrode 22 mit entsprechendem Risiko einer Verletzung vermieden werden.

Auch in anderen Anwendungen sollte eine geeignete relative Positionierung der Sondenspitze zu dem Endoskopende sichergestellt werden, um bspw. eine effiziente endoskopische Behandlung oder Sitzung zu ermöglichen oder einen unerwünschten Kontakt einer Optik oder eines Instrumentes an der Sondenspitze mit dem Gewebe zu vermeiden.

Um die gewünschte relative Positionierung des Arbeitsinstrumentes 3 in Bezug auf das Endoskop 2 zu ermöglichen und den Lagezustand der aktiven Elektrode 22 zu überwachen, ist eine Positionserkennungseinrichtung 27 vorgesehen. Erneut bezugnehmend auf die Figuren 2 und 3 weist die Positionserkennungseinrichtung 27 einen Lichtleiter 28 auf, der an dem Arbeitsinstrument 3 verankert und eingerichtet ist, um Licht, das das Arbeitsinstrument 3 in der Nähe seines distalen Endes 14 umgibt, aufzunehmen, wobei über das durch den Lichtleiter 28 aufgenommene Licht die relative Position des Arbeitsinstrumentes 3 zu dem Endoskop 2 bestimmbar ist.

Der Lichtleiter 28 ist an dem Arbeitsinstrument 3 derart fest verankert, dass er gemeinsam mit dem Arbeitsinstrument 3 in den Arbeitskanal 7 des Endoskops 1 einführbar und darin verschiebbar ist. Dabei kann sich der Lichtleiter ausgehend von einer Stelle in der Nähe des distalen Endes 14 des Arbeitsinstrumentes 3 bis zu dem proximalen Ende 16 erstrecken, um das von der Umgebung aufgenommene Licht zur Auswertung zu dem proximalen Ende des Arbeitsinstrumentes 3 zu leiten. Der Lichtleiter 28 kann eine beliebige optische Faser, z.B. Glasfaser oder vorzugsweise eine polymere optische Faser, aufweisen. Es sind relativ kostengünstige optische Fasern erhältlich, die sich für die vorliegende Funktion der Positionserkennung eignen, für die keine höchste qualitative, absolut verlustlose Lichterfassung und -übertragung erforderlich ist.

Erneut bezugnehmend auf die Figuren 2 und 3 ist der Lichtleiter 28 in der dargestellten Ausführungsform weitgehend in dem inneren Kanal 26 des Arbeitsinstrumentes 3 untergebracht. Der Lichtleiter 28 weist ein erstes Ende 29, das an dem Rohr 23 des Instrumentes 3 befestigt ist, ein zweites Ende 31, das über das proximale Ende 16 des Rohrs 23 herausgeführt ist, und einen dazwischen verlaufenden Lichtleitermittelabschnitt 32 auf, der im Wesentlichen in dem inneren Kanal 26 untergebracht ist. Das erste Ende 29 des Lichtleiters 28 ragt durch die Wand 24 des Rohrs 23 hindurch und weist eine Stirnfläche 33 auf, die in Bezug auf die hier nicht näher dargestellte Längsachse des Arbeitsinstrumentes 3 radial oder seitlich nach außen ausgerichtet ist. Die Stirnfläche 33 bildet eine Lichteinleitfläche, um Außenlicht an der Außenseite des Rohrs 23 zu erfassen und in den Lichtleiter 28 zu leiten. Über den Lichtleitermittelabschnitt 32 wird das erfasste Licht dann bis zu dem zweiten Ende 31 des Lichtleiters 28 übertragen.

Das erste Ende 29 des Lichtleiters 28 ist in einer Öffnung 34, die in dem flexiblen Rohr 23 eingearbeitet ist, formpassend aufgenommen und kann darin zusätzlich bspw. durch Kleben gesichert sein. Die als Lichteinleitfläche dienende Stirnfläche 33 des Lichtleiters 28 schließt vorzugsweise mit der Außenseite des Rohrs 23 im Wesentlichen bündig ab. Das Rohr 23 selbst besteht aus einem lichtundurchlässigen Material.

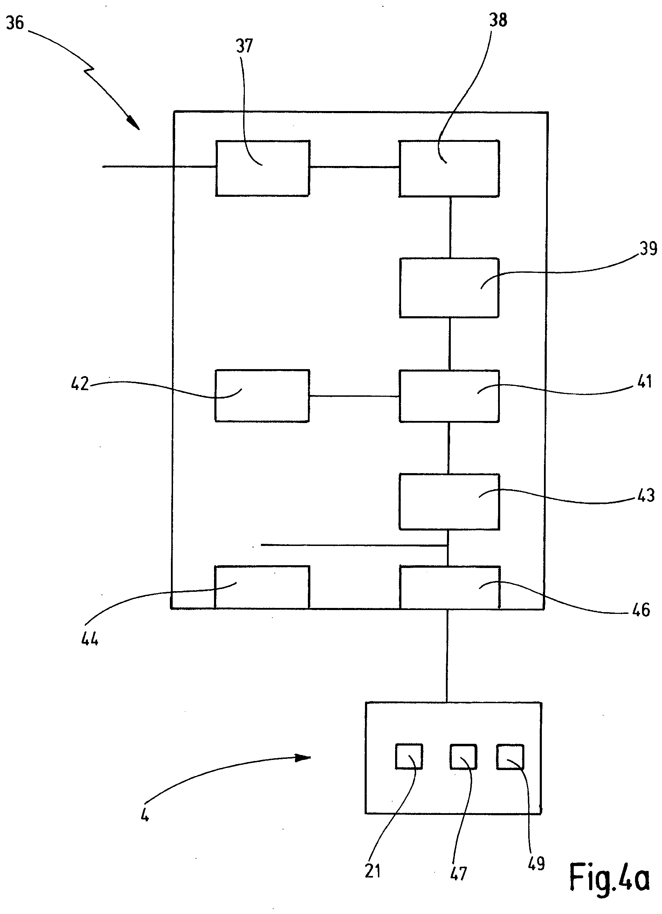

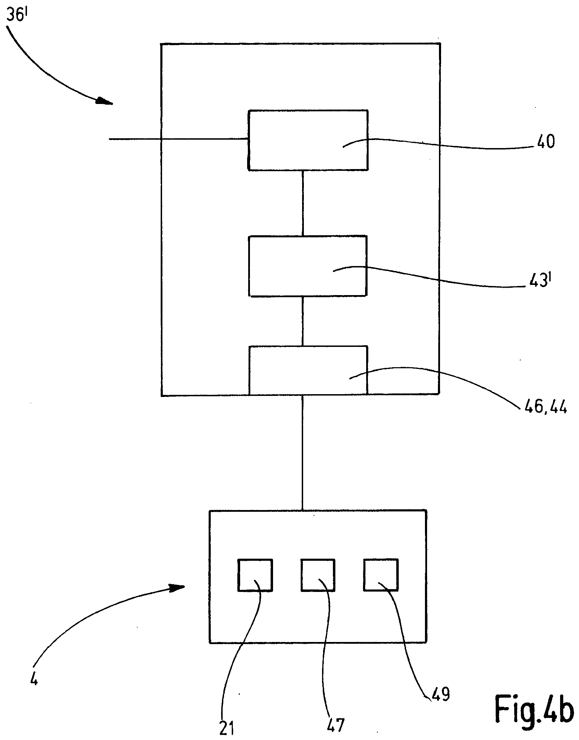

Das zweite Ende 31 des Lichtleiters 28 ist mit einer Auswerteeinrichtung 36 verbunden, die an dem proximalen Ende 16 des Arbeitsinstrumentes 3 anschließbar oder angeschlossen und dazu eingerichtet, das durch den Lichtleiter 28 aufgenommene Licht zu empfangen und auszuwerten, um die relative Position des Arbeitsinstrumentes 3 zu ermitteln. Ausführungsformen der Auswerteeinrichtung 36 sind in vereinfachten Blockschaltbilddarstellungen in den Figuren 4a und 4b näher veranschaulicht.

Bezugnehmend auf Figur 4a kann die Auswerteeinrichtung in einer ersten Ausführungsform eine Spektrometereinheit 37, einen elektro-optischen Wandler 38, eine Signalkonditionierungseinheit 39, eine Spektrumanalyseeinheit 41, einen Speicher 42, eine Detektoreinheit 43, eine Ausgabeeinheit 44 und eine Schnittstelle 46 aufweisen. Wenngleich die Einheiten 37-46 hier als gesonderte Blöcke dargestellt sind, bilden sie lediglich Funktionseinheiten, die in einer oder mehreren physikalischen Einheiten enthalten, auf unterschiedliche Einheiten verteilt, in Hardware und/oder Software realisiert und wenigstens zum Teil durch eine Logik verkörpert sein können, die in Form eines Codes zur Ausführung auf einem hier nicht näher dargestellten Prozessor implementiert sein kann, um die Funktion der Auswerteeinrichtung 36 zur Auswertung des vom Lichtleiter 28 empfangenen Lichts und zur Erkennung der relativen Position des Instrumentes 3 zu erfüllen.

Die Spektrometereinheit 37 ist mit dem Lichtleiter 28 verbunden, um von diesem das aufgenommene Licht zu empfangen, und teilt das Licht spektral in einzelne Frequenzanteile auf. Die Spektrometereinheit 37 kann bspw. ein lichtbrechendes Prisma oder lichtbeugendes Gitter aufweisen. Das resultierende Licht kann bedarfsweise gefiltert werden, bevor es dem elektro-optischen Wandler 38 zugeführt wird. Der elektro-optische Wandler 38, der auch ein Fotodiodenarray sein kann, wandelt das spektral aufgeteilte Licht in entsprechende elektrische Signale zur nachfolgenden Auswertung und Analyse um, um das Frequenzspektrum des aufgenommenen Lichtsignals zu bestimmen. Die gewonnenen elektrischen Signale können in der Signalkonditionierungseinheit 38 verstärkt, von Störsignalen gefiltert, z.B. tiefpass- oder bandpassgefiltert, und in sonstiger Weise für die weitere Verarbeitung aufbereitet werden.

Die Spektrumanalyseeinheit 41 ist dazu eingerichtet, die elektrischen Signale, die die spektral aufgeteilten Lichtanteile repräsentieren, auszuwerten, um die Spektralanteile des Lichts bzw. die Intensitäten des Lichtes in unterschiedlichen Lichtfrequenzbereichen oder an den bestimmten diskreten Frequenzen zu ermitteln und mit abgespeicherten Referenzspektren zu vergleichen, um anhand des Vergleichs das durch den Lichtleiter aufgenommene Licht zu klassifizieren oder zu identifizieren. Die Referenzspektren können im Voraus bestimmt und in dem Speicher 42 vorab gespeichert werden. Es kann ein Referenzspektrum für das Kaltlicht des Endoskops 3, für das Umgebungslicht eines Arbeitsraums sowie für das aus der Funkenentladung bei der APC-Koagulation resultierende Plasmalicht abgespeichert werden. Alle diese Lichtquellen weisen charakteristische Spektren auf, die sich hinsichtlich des jeweiligen Frequenzbereiches und der Intensitätswerte darin deutlich voneinander unterscheiden. Bspw. wird ein Umgebungslicht eines Arbeitsraums Wellenlängen im Bereich von 300-1100 nm mit einer charakteristischen ersten Intensitätsverteilung aufweisen, während das Arbeitslicht aus einer Kaltlichtquelle des Endoskops im Allgemeinen einen Wellenlängenbereich von 400-700 nm oder Teilbereich darin umfasst und eine zweite charakteristische Intensitätsverteilung aufweist und das vom Plasma emittierte Licht charakteristische Anteile sowohl im sichtbaren Wellenlängenbereich als auch im Bereich von 200-400 nm mit einer anderen, dritten Intensitätsverteilung aufweist.

Eines oder mehrere von dem Umgebungslicht, dem Kaltlicht des Endoskops und/der dem vom Plasma emittierten Licht können auch empirisch erfasst werden, um daraus die zugehörigen Referenzspektren zu bestimmen und abzuspeichern. Alternativ können auch repräsentative Frequenzspektren, die von Herstellern der jeweiligen Leuchten oder Geräte erhalten werden, oder simulierte Lichtspektren als Referenzspektren verwendet werden. Jedenfalls ist die Spektrumanalyseeinheit 41 in der Lage, durch einen Vergleich von Intensitätswerten bestimmter diskreter Frequenzlinien oder Frequenzbereiche in verschiedenen Frequenzbändern festzustellen, ob Licht durch den Lichtleiter 28 aufgenommen wurde oder nicht, und wenn ja, ob es sich dabei um das Kaltlicht der Beleuchtungseinrichtung 12 des Endoskops 2, das Umgebungslicht des Untersuchungs- bzw. Behandlungsraums oder ein Lichtsignal aufgrund der Funkenentladung handelt.

Die Detektoreinheit 43 überwacht die von der Spektrumanalyseeinheit identifizierte Lichtart und ist dazu eingerichtet, bei einem Einschieben des Arbeitsinstrumentes 3 in den Arbeitskanal 7 des Endoskops 2 zu erkennen, wenn die Lichteinleitfläche, insbesondere die Stirnfläche 33 des Lichtleiters 28, der Umgebung des Behandlungsraums ausgesetzt ist, aus der Umgebung in das proximale Ende 13 des Arbeitskanals 7 eintritt oder aus dem Arbeitskanal 7 in einen zu behandelnden Körperbereich heraustritt. Basierend auf dieser Erkennung generiert die Detektoreinheit 43 entsprechende Positionserkennungssignale, die bspw. den Beginn des Einführens des Arbeitsinstrumentes 3 in den Arbeitskanal 7 kennzeichnen oder anzeigen, dass das distale Ende 14 des Instrumentes 3 das distale Ende 8 des Endoskops 2 um den gewünschten Abstand überragt, der der Distanz zwischen dem distalen Ende 14 des Arbeitsinstrumentes 3 und der Lichteinleitfläche 33 entspricht.

Die optionale Ausgabeeinheit 44 kann mit der Detektoreinheit 43 verbunden sein, um von dieser die Positionserkennungssignale zu empfangen und diese in optische, akustische oder taktile Anzeigesignale umsetzen, die von einem Bediener des chirurgischen Arbeitsinstrumentes 3 wahrgenommen werden können. Somit ist der Bediener in der Lage zu erkennen, wenn das Arbeitsinstrument 3 in der gewünschten relativen Arbeitslage in Bezug auf das Endoskop 2 positioniert ist.

Die Detektoreinheit 43 kann alternativ oder zusätzlich über die Schnittstelle 46 mit dem Steuergerät 4 des Endoskopsystems 1 verbunden sein, das die Positionserkennungssignale von der Detektoreinheit 43 empfangen und basierend darauf automatisch geeignete Maßnahmen initiieren, bspw. eine Aktivierungssperre, eine Voraktivierung, eine Parametrisierung und/oder eine Steuerung des Betriebs des Arbeitsinstrumentes 3 und/oder des Endoskops 2 bewirken kann.

Figur 4b zeigt eine weitere, besonders bevorzugte Ausführungsform einer Auswerteeinrichtung 36', die sehr komplexarm realisiert und zu einer stark vereinfachten Auswertung des von dem Lichtleiter 28 aufgenommen Lichtes zur groben Positionsbestimmung eingerichtet sein kann. Insbesondere kann die Auswerteeinrichtung 36' lediglich die Erkennung der Anwesenheit und Abwesenheit des aufgenommenen Lichtes bzw. eine Hell-Dunkel-Unterscheidung ermöglichen. Wie aus Figur 4b ersichtlich, kann die Auswerteeinrichtung 36' in diesem Fall ein Mittel 40 zur Umwandlung des aufgenommenen Lichtsignals in ein elektrisches Signal, insbesondere eine Fotodiode, und eine Detektoreinheit 43' aufweisen, die einen Hell-Dunkel-Detektor bildet. Die Detektoreinheit 43' kann vorzugsweise die Stärke des von der Fotodiode oder dgl. erzeugten elektrischen Signals mit einer vorbestimmten Schwelle vergleichen, um festzustellen, ob das von dem Lichtleiter 28 aufgenommene Licht eine Mindestintensität aufweist oder nicht, und basierend darauf Positionserkennungssignale zu generieren, die kennzeichnen, dass das distale Ende 14 des Arbeitsinstrumentes 3 Licht ausgesetzt ist (Helligkeit) oder nicht (Dunkelheit), also sich z.B. außerhalb oder innerhalb des Arbeitskanals 7 des Endoskops 2 befindet. Somit können auch Hell-dunkel- oder Dunkel-hell-Übergänge erfasst werden. Ein Bediener oder eine übergeordnete Steuerung in dem externen Steuergerät 4 kann dann anhand des momentanen Betriebszustands erkennen, in welcher relativen groben Position sich das Arbeitsinstrument 3 befindet. Eine Differenzierung zwischen den unterschiedlichen Lichtarten und eine genauere Positionsbestimmung sind hier nicht erforderlich. Insofern können hier die Funktionseinheiten 37-41 für die Frequenzspektrumbestimmung und -analyse weggelassen werden. Die in Figur 4b zusätzlich dargestellte Schnittstelle 44, 46 kann im einfachsten Fall eine Anschlussleitung, ein Steckerstift oder eine Steckerbuchse sein.

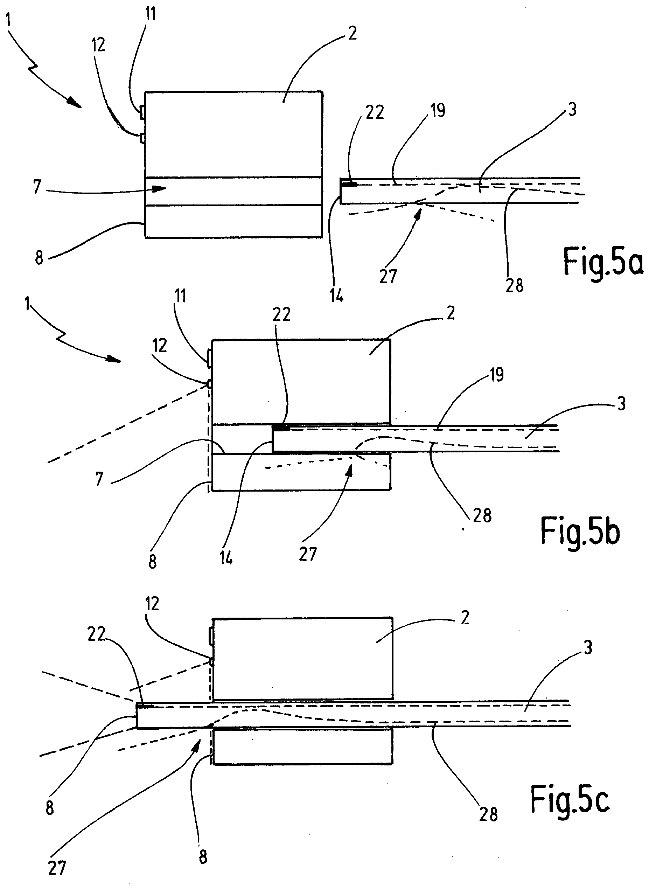

Die Funktionsweise des soweit beschriebenen erfindungsgemäßen Endoskopsystems 1 soll nachstehend unter zusätzlicher Bezugnahme auf die Figuren 5a-5c erläutert werden, in denen die Funktionsabläufe mit den jeweiligen relativen Positionen des Arbeitsinstrumentes 3 zu dem Endoskop 2 gesondert dargestellt sind. Das Endoskopsystem 1 funktioniert wie folgt:
Soll eine Argon-Plasma-Koagulation durchgeführt werden, kann der Bediener über das Steuergerät 4 den gewünschten Modus auswählen, wodurch Komponenten des Endoskopsystems 1, einschließlich der Positionserkennungseinrichtung 27, aktiviert werden können. Die Positionserkennungseinrichtung 27 erkennt über das durch den Lichtleiter 28 aufgenommene Licht nach Auswertung durch die Auswerteeinrichtung 36 bzw. 36', dass sich die Sonde 3 noch außerhalb des Endoskops 2 befindet, wie in Figur 5a veranschaulicht, und dem Umgebungslicht des Behandlungsraums ausgesetzt ist. Die Detektoreinheit 43 bestimmt z.B., dass das ermittelte Frequenzspektrum des aufgenommenen Lichtes mit dem Referenzspektrum des Umgebungslichts zumindest an bestimmten diskreten Frequenzen übereinstimmt, sich jedenfalls deutlich von den Referenzspektren der Kaltlichtquelle des Endoskops 2 und des vom Plasma emittierten Lichtsignals unterscheidet und meldet ein entsprechendes Positionssignal über die Schnittstelle 44 bzw. 46. Alternativ meldet die Hell-Dunkel-Detektoreinheit 43' ein Helligkeitssignal. Soweit hier von Positionssignalen die Rede ist, sollen darunter auch die von der Hell-Dunkel-Detektoreinrichtung 40, 43' generierten Helligkeits- und Dunkelheits-Signale verstanden werden.

Wenn der Bediener anschließend das Arbeitsinstrument 3 in das proximale Ende 13 des Endoskops 2 einführt und die Stirnfläche 33 des Lichtleiters 28 in den Arbeitskanal 7 eintritt, wie in Figur 5b veranschaulicht, sinkt die Intensität des von dem Lichtleiter 28 aufgenommenen Lichtes relativ abrupt ab. Im Inneren des Arbeitskanals 7 herrscht weitgehend absolute Dunkelheit. Das Ausbleiben des aufgenommenen Lichtsignals kann schnell und zuverlässig von der Auswerteeinrichtung 36 bzw. 36' erkannt werden, woraufhin die Detektoreinheit 43 bzw. 43' ein entsprechendes Dunkelheits-Signal an die Ausgabeeinheit 44 und/oder an das Steuergerät 4 ausgeben kann. Das Steuergerät 4 kann daraufhin, sofern nicht bereits geschehen, eine Voraktivierung des Endoskopsystems 1, einschließlich einer Parametrisierung der HF-Generatoreinrichtung 21, Einschaltung der Beleuchtungseinrichtung 12 und Voreinstellung von Steuerventilen für die Gaszuführung, bewirken. In manchen Anwendungen kann die Inertgaszufuhr bereits gestartet werden, sobald erkannt wird, dass das Arbeitsinstrument 3 in den Arbeitskanal 7 eingeführt wird, um während des Einführvorgangs eine Reinhaltung des Arbeitsinstrumentes 3 zu ermöglichen.

Sobald das distale Ende 14 der Sonde 3 so weit aus dem distalen Ende 8 des Endoskops 2 austritt, dass die Stirnfläche 33 des Lichtleiters 28 den Arbeitskanal 7 verlässt, wie in Figur 5c veranschaulicht, erfasst die Stirnfläche 33 das Arbeitslicht, das von der Beleuchtungseinrichtung 12 an dem distalen Ende 8 des Endoskops 2 zur Beleuchtung des Behandlungs- bzw. Operationsbereiches ausgestrahlt wird. Dieses Licht wird von einer geeigneten Kaltlichtquelle 47, die bspw. in dem Steuergerät 4 integriert oder auch eine externe Lichtquelle sein kann, geliefert. Die Auswerteeinrichtung 36 bzw. 36' der Positionserkennungseinrichtung 27 empfängt das Lichtsignal über den Lichtleiter 28 und erkennt den abrupten Wechsel zwischen ausbleibendem und vorliegendem Lichtsignal. Bspw. erkennt der Hell-Dunkel-Detektor 43' den Dunkel-hell-Übergang und meldet wieder ein Helligkeits-Signal. Alternativ kann bei der Auswerteeinrichtung 36 die Spektrumanalyseeinheit 41 das Frequenzspektrum des aufgenommenen Lichtes bestimmen und unter Rückgriff auf in dem Speicher 42 abgespeicherte Referenzspektren feststellen, dass es sich bei dem aufgenommenen Licht um das Arbeitslicht, also das Kaltlicht des Endoskops 2 handelt. Über die Ausgabeeinheit 44 kann dann die Detektoreinheit 43 bzw. 43' das entsprechende Positionserkennungssignal für den Bediener ausgegeben, der folglich erkennen kann, dass die Sondenspitze mit der Elektrode 22 nun die gewünschte Sollposition erreicht hat, so dass das Arbeitsinstrument 3 nicht mehr weiter vorgeschoben werden sollte.

Das Positionserkennungssignal kann alternativ oder zusätzlich über die Schnittstelle 46 zu dem Steuergerät 4 übermittelt werden, das daraufhin die Aktivierungssperre für das elektrische HF-Signal aufheben und auch bereits mit der Steuerung des Betriebs des Arbeitsinstrumentes 3 selbsttätig beginnen kann. Bspw. kann, sofern nicht bereits geschehen, automatisch die Zuführung des zu ionisierenden Gases, z.B. Argon, zu dem Arbeitsinstrument 3 ausgelöst und die Zündung einer Plasmaentladung durch die Elektrode 22 freigegeben werden. Somit kann die HF-Generatoreinrichtung 22 das erforderliche HF-Spannungssignal über die Verbindungsleitung 19 der Elektrode 22 zuführen, um eine Zündung der Plasmaentladung und eine Ionisierung des Gases zu ermöglichen, wonach das erzeugte Argon-plasma als Stromleiter verwendet wird, um den Koagulationsstrom auf das zu behandelnde Gewebe zu übertragen. Über die Optik 11, z.B. eine CCD-Kamera, kann der behandelte Bereich aufgenommen werden, und die Bildsignale können über einen Lichtwellenleiter 48 einer Bildverarbeitungseinheit 49 des Steuergerätes 4 zugeführt werden, um auf einem hier nicht näher veranschaulichten Display dem Bediener angezeigt zu werden.

Sollte der Bediener unerwünscht oder erwünscht das Arbeitsinstrument 3 wieder soweit zurückziehen, dass die Stirnfläche 33 des Lichtleiters 28 in den Arbeitskanal 7 hinein gelangt, wird dies durch die Auswerteeinheit 36 bzw. 36' der Positionserkennungseinrichtung 27 erkannt. Das Steuergerät 4 kann dann automatisch den Betrieb des Arbeitsinstrumentes 3 anhalten und eine Aktivierungssperre bewirken, um die Gas- und/oder HF-Spannungszufuhr zu unterbrechen, so dass die Funkenentladung eingestellt und ein Schaden an dem Endoskop 2 verhindert wird.

Die Aktion eines wiederholten Ein- und Herausschiebens des Arbeitsinstrumentes 3 in den und aus dem Arbeitskanal 7 könnte vom Bediener auch gezielt durchgeführt werden, um die genaue Stelle der gewünschten Arbeitsposition für das Arbeitsinstrument 3 zu identifizieren. Insofern kann die Aktivierung oder Aufhebung der Aktivierungssperre verzögert werden, solange der Bediener noch Einstellungen vornimmt.

Im Rahmen der Erfindung sind zahlreiche Modifikationen möglich. So kann die Auswerteeinrichtung 36 bzw. 36' in einer von dem Arbeitsinstrument 3 gesonderten Geräteeinheit, z.B. in dem Steuergerät 4, aufgenommen sein. Alternativ können Teile, bspw. in Hardware realisierbare Teile der Auswerteeinrichtung 36 bzw. 36' in dem Verbinder 15 untergebracht sein, der die Schnittstelle zum externen Steuergerät 4 bildet, während andere Teile, insbesondere in Firm- oder Software realisierbare Tele der Auswerteeinrichtung 36 bzw. 36' z.B. in dem Steuergerät 4 oder einem anderen externen Gerät implementiert sein können.

In einer besonders bevorzugten Ausführungsform, wie sie in Figur 3 durch Strichlinien angedeutet ist, ist die Auswerteeinrichtung 36' nach Figur 4b mit der durch die Fotodiode 40 und den Hell-Dunkel-Detektor 43' gebildeten Detektoreinrichtung vollständig in dem Verbinder 15 untergebracht, der an dem proximalen Ende 16 des Arbeitsinstrumentes 3 angeordnet ist und dem Anschluss an das Steuergerät 4 dient. Dadurch kann eine äußerst kompakte, weitgehend selbständige Einheit aus dem Arbeitsinstrument 3 und der zugehörigen Positionserkennungseinrichtung 27 bereitgestellt werden, die sich zur Verwendung als Einmalsonde gut eignet, weil die komplexarme, relativ kostengünstige Detektoreinrichtung 40, 43' nach einmaliger Benutzung mit entsorgt werden kann.

In einer weiteren Modifikation kann die in dem Verbinder 15 integrierte Hell-Dunkel-Detektoreinrichtung der Auswerteeinrichtung 36' nach Figur 4b nur die Fotodiode 40 oder ein sonstiges Mittel zur Umwandlung des aufgenommenen Lichts in ein elektrisches Signal enthalten. Das elektrische Signal kann dann zur Schwellenfilterung und/oder Auswertung über die Schnittstelle 46 an das externe Steuergerät 4 übermittelt werden. Die Funktionalität der Hell-Dunkel-Detektoreinheit 43' in Figur 4b kann auf das Steuergerät 4 verlagert werden, was den Aufwand und die Kosten für die in dem Verbinder 15 verbaute Auswerteeinrichtung 36' weiter reduziert.

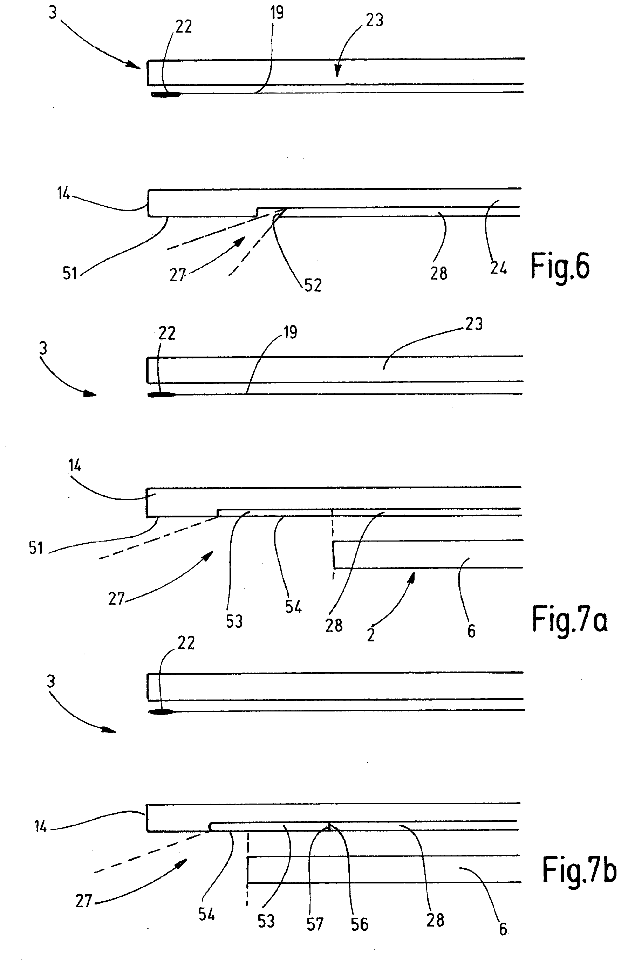

Weitere Ausführungsformen der erfindungsgemäßen Positionserkennungseinrichtung 27 sind in den Figuren 6 und 7a, 7b veranschaulicht. Soweit Übereinstimmung im Aufbau und/oder in der Funktionsweise besteht, wird unter Zugrundelegung gleicher Bezugszeichen auf die vorstehende Beschreibung verwiesen.

Figur 6 zeigt eine Ausführungsform, bei der der Lichtleiter 28 nicht in dem inneren Kanal 26 des Rohrs 23 des Arbeitsinstrumentes 3, sondern an der Außenseite 51 der Wand 24 angeordnet ist. Der Lichtleiter 28 kann eine abgeschrägte Stirnfläche oder Faserfacette 52 aufweisen, die als Lichteinleitfläche dient, um das umgebende Licht an dem distalen Ende 14 des Arbeitsinstrumentes 3 zu erfassen und in den Lichtleitermittelabschnitt 32 einzuleiten, der das Licht zur Auswertung weiter zu dem proximalen Ende 16 des Arbeitsinstrumentes 3 überträgt. Die abgeschrägte Stirnfläche 52 ist sowohl radial oder seitlich nach außen als auch in Längsrichtung des Instrumentes 3 vorzugsweise zu dem distalen Ende 14 hin gerichtet. Der Mittelabschnitt 32 des Lichtleiters 28 erstreckt sich ausgehend von der schrägen Stirnfläche 52 im Wesentlichen geradlinig entlang des Rohrs 23 an dessen Außenseite 51 und kann an der Außenseite 51 bspw. durch Kleben befestigt sein. Prinzipiell ist es auch möglich, den Lichtleiter 28 in der Außenseite 51 des Rohrs 23 im Wesentlichen bündig anzuordnen, indem eine optische Faser bereits bei der Extrusion des Schlauchs 23 des Arbeitsinstrumentes 3 darin eingebettet wird. Unabhängig davon, wie der Lichtleiter 28 an der Außenseite der Wand 24 des Schlauchs 23 angebracht wird, ist die Anbringung an der Außenseite gegenüber der Unterbringung in dem inneren Kanal 26 des Arbeitsinstrumentes 3 erleichtert. Die Funktionsweise der Positionserkennungseinrichtung 27 in der Ausführungsform nach Figur 6 entspricht der im Zusammenhang mit den Figuren 1-5 vorstehend beschriebenen.

Figuren 7a und 7b zeigen eine noch weitere Ausführungsform der Positionserkennungseinrichtung 27 gemäß der Erfindung, wobei hier die Lichteinleitfläche zur Erfassung des das Arbeitsinstrument 3 umgebenden Lichtes durch ein gesondertes Lichteinleitelement, nämlich in dem dargestellten Beispiel durch einen länglichen Glasstab 53 gebildet ist. Der Glasstab 53, der aus Silikatglas oder Acrylglas gebildet sein kann, ist an der Außenseite 51 des Rohrs 23 angeordnet und daran befestigt und/oder darin eingebettet. Jedenfalls weist der längliche Glasstab 53 eine Länge auf, die größer ist als dessen Breite und längs der Längserstreckung des Arbeitsinstrumentes 3 ausgerichtet ist. Die Länge des Glasstabs 53 kann beliebig, vorzugsweise größer als die halbe Auskraglänge des distalen Endes 14 des Arbeitsinstrumentes 3 in der gewünschten Arbeitsposition bzw. als der Abstand zwischen dem distalen Ende 14 des Arbeitsinstrumentes 3 und dem distalen Ende des Glasstabs 53, sein. Die radial oder seitlich nach außen gewandte Außenfläche 54 des Glasstabs 53 dient als die Lichteinleitfläche. Ein von dem distalen Ende 14 des Arbeitsinstrumentes 3 abgewandtes Stirnende 56 des Glasstabs 53 ist mit einem Stirnende 57 des Lichtleiters 28 verbunden, so dass das von dem Glasstab 53 aufgenommene Licht in den Lichtleiter 28 und durch diesen weiter zu dem proximalen Ende 16 des Arbeitsinstrumentes 3 hin übertragen wird.

Die Funktionsweise des Arbeitsinstrumentes 3 und der Positionserkennungseinrichtung 27 in der Ausführungsform gemäß Figur 7a, 7b entspricht weitgehend derjenigen der vorstehend beschriebenen Ausführungsformen, wobei jedoch hier eine genauere Bestimmung der relativen Position des Arbeitsinstrumentes 3 in Bezug auf das Endoskop 2 ermöglicht ist. Bspw. zeigt Figur 7a den Zustand der gewünschten Positionierung des Arbeitsinstrumentes 3, bei der das distale Ende 14 des Arbeitsinstrumentes 3 soweit aus dem Arbeitskanal 7 herausgetreten ist, dass sich die Elektrode 22 in dem gewünschten Abstand zu dem distalen Ende 8 des Endoskops 2 befindet. In diesem Fall ist auch der Glasstab 53 vollständig aus dem Arbeitskanal 7 herausgeschoben, so dass ein intensitätsstarkes Lichtsignal über den Lichtleiter 28 aufgenommen und der Positionserkennungseinrichtung 27 zugeführt wird. Die Auswerteeinrichtung 36 bzw. 36' erkennt das Lichtsignal der maximalen Intensität bspw. anhand einer maximalen von mehreren Schwellen und erzeugt daraufhin ein Positionserkennungssignal, das anzeigt, dass sich das Arbeitsinstrument 3 nun in der gewünschten Arbeitsposition befindet.

In Figur 7b ist ein Zustand dargestellt, bei dem der Abstand des distalen Endes 14 bzw. der Elektrode 22 des Arbeitsinstrumentes 3 zu dem distalen Ende 8 des Endoskops 2 kleiner ist als der gewünschte Abstand. Dieser Zustand kann entweder beim Einschieben des Arbeitsinstrumentes 3 in den Arbeitskanal 7 in Vorbereitung auf eine APC-Koagulation oder sonstige endoskopische Behandlung oder Untersuchung oder im Betrieb vorliegen, wenn der Bediener z.B. aus Versehen das Arbeitsinstrument 3 wenigstens teilweise wieder in den Arbeitskanal 7 zurück einzieht. Das Rohr 6 des Endoskops 2 deckt in diesem Fall wenigstens einen Abschnitt der Außenfläche 54 des Glasstabs 53 ab, so dass über den Glasstab 53 nur eine verringerte Lichtmenge erfasst und in den Lichtleiter 28 eingeleitet wird. Die Auswerteeinrichtung 36 bzw. 36' erkennt die reduzierte Intensität des aufgenommenen Lichtes und kann in diesem Fall bspw. die Aktivierungssperre initiieren oder aufrechterhalten, um zu verhindern, dass bei einer Zündung der Plasmaentladung aufgrund der Nähe der Elektrode 22 zu dem Endoskopende das Endoskop 2 oder dessen Komponenten beschädigt wird bzw. werden.

Es ist ein chirurgisches Arbeitsinstrument 3 offenbart, das in einen Arbeitskanal 7 eines Endoskops 2 eingeführt und darin verschiebbar aufgenommen wird. Eine Einrichtung 27 zur Erkennung der relativen Position des Arbeitsinstrumentes 3 zu dem Endoskop 2 ist dazu eingerichtet, das Erreichen eines distalen Endes 8 des Arbeitskanals 7 durch ein distales Ende 8 des Arbeitsinstrumentes 3 in optischer Weise zu erkennen. Die Positionserkennungseinrichtung 27 weist einen Lichtleiter 28 auf, der an dem Arbeitsinstrument 3 verankert und eingerichtet ist, um Licht, das das Arbeitsinstrument 3 in der Nähe seines distalen Endes 14 umgibt, aufzunehmen, wobei über das durch den Lichtleiter 28 aufgenommene Licht die relative Position des Lichtleiters 28 und somit des Arbeitsinstrumentes 3 zu dem Endoskop 2 bestimmbar ist.

## Patentansprüche

1. Chirurgisches Arbeitsinstrument (3) zur Einführung in einen Arbeitskanal (7) eines Endoskops (2) mit einer Einrichtung (27) zur Erkennung der relativen Position des Arbeitsinstrumentes (3) zu dem Endoskop (2), wobei die Positionserkennungseinrichtung (27) einen Lichtleiter (28) aufweist, der an dem Arbeitsinstrument (3) verankert und eingerichtet ist, um Licht, das das Arbeitsinstrument (3) in der Nähe seines distalen Endes (14) umgibt, aufzunehmen, wobei über das durch den Lichtleiter (28) aufgenommene Licht die relative Position des Arbeitsinstrumentes (3) zu dem Endoskop (2) bestimmbar ist.

2. Chirurgisches Arbeitsinstrument (3) nach Anspruch 1, wobei der Lichtleiter (28) eine optische Faser, insbesondere eine Glasfaser oder auf Kunststoff basierende optische Faser, aufweist, die an dem Arbeitsinstrument (3) derart verankert ist, dass sie beim Einführen des Arbeitsinstrumentes (3) in den Arbeitskanal (7) des Endoskops (2) gemeinsam mit diesem eingeschoben und darin verschoben wird.

3. Chirurgisches Arbeitsinstrument (3) nach Anspruch 1 oder 2, wobei sich der Lichtleiter (28) ausgehend von einer Stelle in der Nähe des distalen Endes (14) des Arbeitsinstrumentes (3) bis zu einem proximalen Ende (16) des Arbeitsinstrumentes (3) erstreckt, um das durch den Lichtleiter (28) aufgenommene Licht zur Auswertung zu dem proximalen Ende (16) des Arbeitsinstrumentes (3) zu übertragen.

4. Chirurgisches Arbeitsinstrument (3) nach einem der vorhergehenden Ansprüche, wobei das Arbeitsinstrument (3) ein Rohr (23) aufweist, wobei die Positionserkennungseinrichtung (27) eine Lichteinleitfläche (33, 52, 54) aufweist, die an der Außenseite des Rohrs (23) angeordnet und zur seitlichen Lichterfassung des das distale Ende (14) des Arbeitsinstrumentes (3) umgebenden Lichtes eingerichtet ist.

5. Chirurgisches Arbeitsinstrument (3) nach Anspruch 4, wobei die Lichteinleitfläche durch eine aus dem Rohr (23) seitlich herausgeführte Stirnfläche (33) des Lichtleiters (28) gebildet ist, wobei sich der Lichtleiter (28) im Wesentlichen im Inneren des Rohrs (23), entlang dessen Längserstreckung erstreckt.

6. Chirurgisches Arbeitsinstrument (3) nach Anspruch 4, wobei die Lichteinleitfläche durch eine abgeschrägte Stirnfläche (52) des Lichtleiters (28) gebildet ist, wobei der Lichtleiter (28) an der Außenseite des Rohrs (23) angebracht ist und sich entlang dessen Längserstreckung erstreckt.

7. Chirurgisches Arbeitsinstrument (3) nach Anspruch 4, wobei die Lichteinleitfläche durch eine Außenfläche (54) eines länglichen Glasstabs (53) gebildet ist, der an der Außenseite des Rohrs (23) derart angeordnet ist, dass seine Längserstreckung in der Längsrichtung des Arbeitsinstrumentes (3) ausgerichtet ist, und dessen von dem distalen Ende (14) des Arbeitsinstrumentes (3) abgewandtes Stirnende (56) mit dem Lichtleiter (28) verbunden ist.

8. Chirurgisches Arbeitsinstrument (3) nach einem der vorhergehenden Ansprüche, das ferner eine Auswerteeinrichtung (36, 36') aufweist, die an dem proximalen Ende (13) des Arbeitsinstrumentes (3) angeschlossen und eingerichtet ist, um das durch den Lichtleiter (28) aufgenommene Licht zu empfangen und auszuwerten.

9. Chirurgisches Arbeitsinstrument (3) nach Anspruch 8, wobei die Auswerteeinrichtung (36) eine optische Spektrometereinheit (37) aufweist, die eingerichtet ist, um das aufgenommene Licht in seine spektralen Bestandteile aufzuteilen.

10. Chirurgisches Arbeitsinstrument (3) nach Anspruch 9, wobei die Auswerteeinrichtung (36) eine Spektrumanalyseeinheit (41) aufweist, die eingerichtet ist, um die ermittelte Intensität des Lichts über einem Bereich von Lichtfrequenzen oder in bestimmen diskreten Frequenzen mit abgespeicherten Referenzspektren zu vergleichen und anhand des Vergleichs das durch den Lichtleiter (28) aufgenommene Licht zu identifizieren, wobei die Spektrumanalyseeinheit (41) vorzugsweise eingerichtet ist, um wenigstens zwischen Behandlungsraum-Umgebungslicht, Dunkelheit und Behandlungslicht aus dem Endoskop (2) zu differenzieren.

11. Chirurgisches Arbeitsinstrument (3) nach einem der Ansprüche 8-10, soweit auf einen der Ansprüche 4-7 rückbezogen, wobei die Auswerteeinrichtung (36) eine Detektoreinheit (43) aufweist, die eingerichtet ist, um beim Einschieben des Arbeitsinstrumentes (3) in den Arbeitskanal (7) zu erkennen, wenn die Lichteinleitfläche (33, 52, 54) der Umgebung eines Behandlungsraums ausgesetzt ist, aus der Umgebung in den Arbeitskanal (7) eintritt oder aus dem Arbeitskanal (7) in einen zu behandelnden Körperbereich heraustritt, und basierend darauf entsprechende Positionserkennungssignale zu generieren.

12. Chirurgisches Arbeitsinstrument (3) nach Anspruch 8, wobei die Auswerteeinrichtung (36') eine Detektoreinrichtung (40, 43') aufweist, die eingerichtet ist, um zu erkennen, ob von dem Lichtleiter (28) Licht einer Mindestintensität aufgenommen wird oder nicht und basierend darauf entsprechende Positionserkennungssignale zu generieren.

13. Chirurgisches Arbeitsinstrument (3) nach Anspruch 11 oder 12, wobei die Auswerteeinrichtung (36, 36') in einem Verbinder (15) integriert ist, der an einem proximalen Ende (16) des Arbeitsinstrumentes (3) angeordnet und zum Anschluss an ein externes Steuergerät (4) vorgesehen ist.

14. Chirurgisches Arbeitsinstrument (3) nach einem der Ansprüche 11-13, wobei die Auswerteeinrichtung (36, 36') mit einer Steuerung (4) für das Arbeitsinstrument (3) und/oder das Endoskop (2) verbindbar ist, die eingerichtet ist, um basierend auf den Positionserkennungssignalen automatisch eine oder mehrere der folgenden Maßnahmen zu bewirken: Freigeben und Sperren der Aktivierung eines über das Arbeitsinstrument (3) zugeführten elektrischen Signals oder eines Gasflusses durch das Arbeitsinstrument (3), Parametrisierung und/oder Steuerung des Betriebs des Arbeitsinstrumentes (3) und/oder des Endoskops (2) in der gewünschten Arbeitsposition.

15. Chirurgisches Arbeitsinstrument (3) nach einem der vorhergehenden Ansprüche, das eine Koagulationssonde (3) zur HF-Koagulation biologischer Gewebe mittels eines flexiblen Endoskops (2) ist, wobei die Koagulationssonde (3) ein Rohr (23) in Form eines flexiblen Schlauchs aus einem elektrisch nicht leitenden Material, insbesondere aus PTFE, durch den ein inertes Gas, insbesondere Argon, zuführbar ist, und eine am distalen Ende (14) des Rohrs (23) angeordnete Elektrode (22) aufweist, der über eine Verbindungsleitung (19) ein HF-Koagulationsstrom zur Ionisierung des Gases zuführbar ist.
